(19) Europäisches Patentamt

European Patent Office

Office européen des brevets

(11) Veröffentlichungsnummer : **0 453 603 B1**

## (12) EUROPÄISCHE PATENTSCHRIFT

(45) Veröffentlichungstag der Patentschrift :
**06.10.93 Patentblatt 93/40**

(51) Int. Cl.$^5$ : **A61K 31/54, A61K 9/107**

(21) Anmeldenummer : **90107992.1**

(22) Anmeldetag : **26.04.90**

(54) **Piroxicam enthaltende pharmazeutische Zusammensetzung zur topischen Anwendung.**

(43) Veröffentlichungstag der Anmeldung :
**30.10.91 Patentblatt 91/44**

(45) Bekanntmachung des Hinweises auf die
Patenterteilung :
**06.10.93 Patentblatt 93/40**

(84) Benannte Vertragsstaaten :
**AT BE CH DE DK ES FR GB GR IT LI LU NL SE**

(56) Entgegenhaltungen :
**EP-A- 0 101 178**
**EP-A- 0 173 990**
**EP-A- 0 179 430**
**EP-A- 0 313 303**

(73) Patentinhaber : **SAGITTA ARZNEIMITTEL GMBH**
**Frühlingstrasse 7**
**D-83620 Feldkirchen-Westerham (DE)**

(72) Erfinder : **Dölling, Dr. Reinhold**
**Bachweg 8**
**D-8152 Feldkirchen-Westerham (DE)**
Erfinder : **Johann, Uwe Nikolaus**
**Gänselieselstr. 42**
**D-8000 München 83 (DE)**

(74) Vertreter : **Hansen, Bernd, Dr. Dipl.-Chem. et al**
**Hoffmann, Eitle & Partner Patent- und**
**Rechtsanwälte, Postfach 81 04 20**
**D-81904 München (DE)**

EP 0 453 603 B1

**Beschreibung**

Die Erfindung betrifft eine Piroxicam enthaltende pharmazeutische Zusammensetzung zur topischen Anwendung bei Erkrankungen und Verletzungen des Bewegungsapparates (Sportverletzungen, rheumatischer Formenkreis).

Piroxicam (4-Hydroxy-2-methyl-N-2-pyridyl-2H-1,2-benzothiazin-3-carboxamid-1,1-dioxid), eine zur Gruppe der Oxicame (Dihydrothiazinderivate) gehörende Substanz, ist als nicht-stereoidales Antirheumatikum/Antiphlogistikum (NSAR) und Schmerzmittel, z.B. bei Verletzungen und bei Zahnextraktionen, seit vielen Jahren bekannt (vgl. DE-PS 1943265); trotz seiner hohen Wirksamkeit und lange andauernden Wirkung ist seine orale Anwendung aber von den NSAR eigenen Nebenwirkungen, wie Magen-Darmblutungen, Herz- und Nierenfunktionsschädigungen, begleitet (vgl. z.B. Pharmazie in unserer Zeit 13 (1984) 177, 185).

Zur Vermeidung oder Verringerung von Nebenwirkungen ist es deshalb wünschenswert, die orale Applikation durch eine topische Applikation zu ergänzen, insbesondere bei örtlich lokalisierten Verletzungen oder Erkrankungen des Bewegungsapparates.

Für eine topische Anwendung, d.h. Abgabe an und durch die Haut über einen längeren Zeitraum, wurden medizinische Pflaster vorgeschlagen, die als Wirkstoff ein Antiphlogistikum enthalten (vgl. DE-A-3344691, 3347277, 3347278).

Die EP-A-0331382 beschreibt Zusammensetzungen zur transdermalen Applikation mit einem gesteigerten transdermalen Fluß, die neben dem Wirkstoff, z.B. Piroxicam, und einem wässerigen Lösungsmittelsystem einen Penetrationsförderer aus der Gruppe der 1-Alkylazacycloheptan-2-one enthalten.

Aus der japanischen veröffentlichten Patentanmeldung JP 5913714 (8413714) sind Piroxicam enthaltende antiinflammatorisch und analgetisch wirkende Zusammensetzungen bekannt, z.B. in Form einer Salbe mit einer O/W-Salbengrundlage, die Carboxyvinylpolymer, Ethanol, Propylenglycol, Diethanolamin, 2-Hydroxyethyl-celluloseether, Polyvinylpyrrolidon und Wasser enthält.

Die EP-B-0101178 beschreibt ein topisches, entzündungshemmendes Mittel in Gelsalbenform in einem wässerigen System, das eine wirksame, entzündungshemmende Menge Piroxicam, 10 bis 50 Gew. % eines Niederalkanols, eine gelbildende Menge Carboxyvinylpolymer, 5 bis 40 Gew. % eines mehrwertigen Alkohols und eine das Piroxicam löslichmachende Menge von wenigstens einem Alkanolamin in Wasser enthält.

Solche Formulierungen nicht-stereoidaler Antirheumatika, wie z.B. Piroxicam, zur topischen Anwendung in hydrophilen Grundlagen, wie z.B. die vorstehend genannte Öl-in-Wasser-Gelsalbenform, oder Gele, die das Piroxicam in Propylenglycol, Ethyl- und Isopropylalkohol enthalten, besitzen den Vorteil, daß sie als mit Wasser verdünnbare Systeme leicht abwaschbar sind; außerdem sind sie geruchlos und nicht fettend.

Piroxicam bildet ein Monohydrat mit zitronengelber Farbe. Dieses Hydrat ist schwerer löslich als Piroxicam, sodaß wässerige Lösungen von Piroxicam im pH-Bereich von ca. 2-8 als metastabil in der Hinsicht anzusehen sind, daß aus ihnen gelbes Piroxicam-Monohydrat auskristallisieren kann. Die bekannten, Piroxicam enthaltenden Formulierungen in hydrophilen Grundlagen, wie z.B. zur topischen Anwendung geeignete O/W-Cremes besitzen daher den Nachteil, daß sie sich während einer längeren Aufbewahrungszeit aufgrund der Eigenschaft von Piroxicam, sich in das gelbe Monohydrat umzuwandeln, gelbverfärben. Aufgrund dieser Instabilität, und auch wegen des durch die Verfärbung hervorgerufenen unschönen optischen Erscheinungsbildes werden diese Formulierungen vom Verbraucher häufig abgelehnt.

Die EP-A-0179430 beschreibt eine Piroxicam enthaltende Zubereitung, in der das gelbe Piroxicammonohydrat in einer O/W-Creme eingearbeitet ist.

Formulierungen in hydrophilen Grundlagen besitzen in der Regel die Eigenschaft, daß der Wirkstoff gut an die Haut abgegeben wird.

Die Formulierungen auf der Basis hydrophiler Grundlagen zur topischen Anwendung, wie Gele oder O/W-Cremes, besitzen allerdings den Nachteil, daß sie zu einer Austrocknung der Haut führen.

Aufgabe der Erfindung war es, eine Piroxicam enthaltende pharmazeutische Zusammensetzung zur topischen Anwendung bereitzustellen, die die vorstehend genannten Nachteile (Verfärbung aufgrund von Instabilität, Austrocknen der Haut) nicht aufweist, eine gute Stabilität über einen längeren Zeitraum besitzt, und die sich leicht zur äußerlichen Anwendung auf der Haut eignet.

Es wurde nun gefunden, daß diese Aufgabe mit einer Zusammensetzung gelöst werden kann, die den Wirkstoff in einem lipophilen Wasser-in-Öl (W/O)-System enthält.

Gegenstand der Erfindung ist deshalb eine pharmazeutische Zusammensetzung nach Anspruch 1 zur topischen Anwendung auf der Grundlage einer Wasser-in-Öl(W/O)-Emulsion, die dadurch gekennzeichnet ist, daß sie in der Ölphase als Wirksubstanz Piroxicam enthält, und gegebenenfalls weitere Mittel zur Stabilisierung, Konservierung und/oder übliche pharmazeutische Hilfs- und/oder Trägerstoffe.

Zweckmäßige Ausgestaltungen dieser Zusammensetzung sind Gegenstand der Ansprüche 2 bis 20.

Die erfindungsgemäße Zusammensetzung auf der Grundlage einer Wasser-in-Öl-Emulsion enthält das Piroxicam in der Fettphase; durch direkte Einarbeitung in die Fettphase ist es auf diese Weise möglich, das Piroxicam rasch vom Wasser abzutrennen, wodurch sich eine Zubereitung mit einer hervorragenden Langzeitstabilität ergibt. Bei einer im Vergleich zu bisher gebräuchlichen Piroxicam enthaltenden Zubereitungen zur topischen Anwendung besitzen die erfindungsgemäßen Zubereitungen eine zumindest gleich gute antirheumtische /antiinflammatorische Wirksamkeit; durch Einarbeitung von penetrationsfördernden Stoffen läßt sich die Hautresorption optimieren. Die erfindungsgemäßen pharmazeutischen Zubereitungen, z.B. in Form einer Creme, zeigen aufgrund ihrer hervorragenden Langzeitstabilität keine optische Veränderung, wie z.B. Gelbverfärbung. Sie verursachen auch bei einer längeren Applikation keine Schuppenbildung oder allergische Erscheinungen. Die hautpflegenden Eigenschaften sind in der Regel denen eines O/W-Systems überlegen.

Die erfindungsgemäße Zusammensetzung auf der Basis einer Wasser-in-Öl-Emulsion kann in Form einer Salbe, Creme, Paste oder Emulsion vorliegen, wobei der Übergang zwischen diesen Formen fließend sein kann; gemäß der üblichen Definitionen enthält die erfindungsgemäße Zusammensetzung, wenn sie in Form einer Paste vorliegt, z.B. auch noch pulverförmige Bestandteile, z.B. geeignete pharmazeutische Hilfs- und/oder Zusatzstoffe (zur Definition und zur Unterscheidung der vorstehend genannten Begriffe vergleiche Römpp's Chemie Lexikon, 8. neubearbeitete Auflage, 1979, Seite 3661).

Als die Fettphase aufbauende Bestandteile können die für die erfindungsgemäßen Arzneiformen (Salben, Pasten, Emulsionen und insbesondere Cremes) üblichen Fettphasenkomponenten, und insbesondere die für die Herstellung von Dermatika üblichen Komponenten verwendet werden. Als Hauptkomponente für die Herstellung der Fettphasengrundlage (Salbengrundlage) wird erfindungsgemäß bevorzugt Polyethylenwachs-Dispersion auf der Basis von Polyethylen und Mineralöl eingesetzt, z.B. Polycos 20 (von der Firma Croda GmbH, 4054 Nettetal). Weitere erfindungsgemäß bevorzugte Fettphasenkomponenten (als Ölkomponenten) sind Triglyceride, vorzugsweise mittelkettige C 8 bis C 10-Triglyceride, wie z.B. Miglyol®812 (von Dynamit Nobel, Troisdorf) und/oder 2-Octyl-dodecanol. Vorzugsweise enthält die erfindungsgemäße Zusammensetzung ein Gemisch von Polyethylenwachs-Dispersion und mittelkettigen Triglyceriden. Daneben enthält die Fettphase (Ölphase) noch weitere übliche Komponenten, wie Emulgatoren, Stabilisatoren, Antioxidantien, Penetrationsförderer, Penetrationsbeschleuniger, Lösungsvermittler, Konservierungsmittel, Emulsionsstabilisatoren usw.

Als Emulgatoren werden erfindungsgemäß übliche W/O-Emulgatoren eingesetzt, vorzugsweise nichtionogene Emulgatoren, wie z.B. höhere Fettalkohole und Sterinalkohole, Partialfettsäureester mehrwertiger Alkohole, Partialfettsäureester von Sorbitan, Sorbitolether des Polyoxyethylens, Fettalkoholether des Polyoxyethylens, Fettsäureester des Polyglycerols; die Auswahl der geeigneten Emulgatoren richtet sich dabei insbesondere nach dem HLB-Wert (Hydrophylic Lipophylic Balance), wobei im wesentlichen HLB-Werte von 3 bis 10, insbesondere von 3 bis 6, wie sie typischerweise in W/O-Emulsionen verwendet werden, eingesetzt werden (vgl. R. Voigt, Lehrbuch der Pharmazeutischen Technologie, Verlag Chemie, Weinheim-New York 1979, Seiten 358 bis 366; H. Sucker, P. Fuchs und P. Speiser, Pharmazeutische Technologie, Georg Thieme Verlag, Stuttgart 1978, Seiten 300 bis 305).

Besonders bevorzugt wird erfindungsgemäß als Emulgator Glycerin-Sorbitan-Oleostearat (ein Gemisch von Partialestern des Glycerins und Sorbits einschließlich seiner Anhydride mit Oleostearinsäure, die wechselnde Mengen anderer Fettsäuren enthalten kann), z.B. Arlacel®481 (Firma ICI).

Als Stabilisator/Antioxidans wird erfindungsgemäß bevorzugt Butylhydroxyanisol (z.B. Embanox® BHA) eingesetzt; als Penetrationsförderer, der eine dermatophile Gleitschiene für lipoidlösliche Wirkstoffe bereitstellen sollen, wird vorzugsweise 2-Octyldodecanol, z.B. Eutanol®G der Firma Henkel, Düsseldorf) eingesetzt, und/oder Penetrationsbeschleuniger zur dermalen und transdermalen Therapie [vgl. z.B. J. Petersen-Lehman, Pharm. Ztd. 134 (1989) 1031 bis 1032], wie z.B. Propylenglycol und/oder mittelkettige Triglyceride, wie z.B.Miglyol®812.

Als Konservierungsmittel wird vorzugsweise Sorbinsäure eingesetzt, und insbesondere eine Mischung von Sorbinsäure und Propylenglycol. Als Emulsionsstabilisator wird vorzugsweise Magnesiumsulfat-Heptahydrat eingesetzt [vgl. z.B. H.G. Wolf, Pharm. Ztg. 133 (1988) 32].

Neben den vorstehend genannten Komponenten können die erfindungsgemäßen Zusammensetzungen noch weitere Bestandteile enthalten, z.B. Mittel zur pH-Wert-Einstellung, Lichtschutzmittel, Schönungsmittel, Farbstoffe, Feuchthaltemittel, Parfümöle und/oder weitere für derartige Arzneiformel übliche pharmazeutische Hilfs- und/oder Trägerstoffe. Die Aufbewahrung der erfindungsgemäßen Zusammensetzungen erfolgt zweckmäßigerweise in dicht verschlossenen Behältern, sowie kühl- und lichtgeschützt.

Der Gehalt an Piroxicam richtet sich insbesondere nach der Arzneiform, in der die erfindungsgemäßen pharmazeutischen Zusammensetzungen auf der Grundlage einer W/O-Emulsion vorliegen; in der Regel enthalten die erfindungsgemäßen Zusammensetzungen das Piroxicam in einer Menge von 0.1 bis 10 Gew. %, vorzugsweise von 0.2 bis 5 Gew. %, und insbesondere in einer Menge von 0.5 bis 1 Gew.%. Besonders bevorzugte Ausführungsformen der erfindungsgemäßen Zusammensetzungen sind W/O-Cremes mit einem Gehalt von 0.5 bis 1 Gew. % Piroxicam. Gegebenenfalls können die Zusammensetzungen neben dem eigentlichen Wirkstoff Piroxicam auch noch weitere pharmazeutische Wirkstoffe, die mit Piroxicam im Rahmen der Applikation verträglich sind, enthalten, z.B. weitere Wirkstoffe zur Therapie von Hauterkrankungen (Dermatosen), Antibiotika, Sulfonamide, Desinfektionsmittel, Corticoide, Hautpflegemittel und/oder durchblutungsfördernde (hyperämisierende) Mittel, wie z.B. Nicotinsäureester (z.B. Benzylnicotinat), Salicylsäureester (z.B. Bornylsalicylat), Extractum Capsici fructus, ätherische Öle (z.B. Fichtennadelöl).

Das Verhältnis Ölphase/Wasserphase, sowie die Art und die Menge der einzelnen Komponenten der Öl- und Wasserphase sind insbesondere abhängig von der Art (Arzneiform) der erfindungsgemäßen Zusammensetzung, aber auch von der Wirkstoffmenge und der Art der beabsichtigten Applikation (z.B. für kurzfristige Anwendung bei Verletzungen, oder zur längerfristigen Anwendung bei rheumatischen Erscheinungsformen). Die Fettphase (Ölphase) der erfindungsgemäßen Zusammensetzungen umfaßt vorzugsweise ein Gemisch aus Polyethylenwachs-Dispersion (als Salbengrundlage), Glycerin-Sorbitan-Oleostearat (als Emulgator), 2-Octyl-dodecanol und/oder mittelkettigen Triglyceriden, vorzugsweise mit 8 bis 10 Kohlenstoffatomen (als Ölkomponenten), und Butylhydroxyanisol (als Antioxidans). Die erfindungsgemäßen Zusammensetzungen enthalten diese Bestandteile vorzugsweise in den folgenden Mengen: 5 bis 30 % Salbengrundlage, 5 bis 25 % Emulgator, jeweils 0.5 bis 15 % einer oder mehrerer Ölkomponenten, 0.005 bis 0.15 % Antioxidans (die Mengenangaben beziehen sich auf Gew. %, bezogen auf die Gesamtzusammensetzung).

Vorzugsweise liegt die erfindungsgemäße Zusammensetzung in Form einer W/O-Creme vor. Für diesen Fall beträgt das Gewichtsverhältnis von Ölphase zu Wasserphase zweckmäßigerweise 3 zu 2.

Die Wasserphase enthält vorzugsweise Propylenglycol (als Feuchthaltemittel, Penetrationsverstärker und Konservierungsmittel), Sorbinsäure (als Konservierungsmittel), Magnesiumsulfat-Heptahydrat (als Stabilisator), gegebenenfalls Parfümöl und/oder weitere für derartige Zusammensetzungen übliche pharmazeutische Träger- und/oder Zusatzstoffe. Die Wasserphase enthält die Bestandteile vorzugsweise in den folgenden Mengen: 1 bis 20 % Feuchthaltemittel/Penetrationsverstärker, 0.01 bis 0.2% Konservierungsmittel, 0.25 bis 1.0 % Stabilisatoren und 20 bis 65 % gereinigtes Wasser (in Gew. %, bezogen auf die Gesamtzusammensetzung).

Die vorstehend für die einzelnen Komponenten der erfindungsgemäßen Zusammensetzungen genannten Substanzen können aber auch durch andere für derartige Zusammensetzungen übliche Substanzen ersetzt oder ergänzt werden, insbesondere durch eine oder mehrere der folgenden Substanzen:

Als Salbengrundlage: Wollwachs, Polyethylen mit Paraffinöl, Cetylpalmitat, gesättigte Triglyceride, Oleogel DAB9, weißes Vaselin, gelbes Vaselin, Ozokerit, eine Mischung aus festem Paraffin (Mikrowachs) und flüssigem Paraffin; als Emulgator: W/O-Emulgatoren mit HLB-Werten von 3 bis 10, insbesondere von 3 bis 6, wie in erster Linie Mischungen aus Glycerin-Fettsäureestern (z.B. Glycerinmonostearat, -palmitat, -laurat, -myristat und/oder -oleat), bzw. Glycerinmonodifettsäureestern (z.B. Glycerinmonodistearat) bzw. Glycerindifettsäureester (z.B. Glycerindioleat, Glycerindistearat) mit Sorbitanfettsäureestern (z.B. Sorbitanmonooleat, -sesquioleat, -trioleat, -stearat, -laurat, -myristat, -palmitat und/oder -isostearat); für die Ölkomponenten: gesättigte und ungesättigte Fettalkohole mit $C_{12}$ - $C_{18}$-Kettenlängen, Ölsäureoleylester, Polyol-Fettsäureester, Isooctylstearat, Hexyllaurat, Di-n-butyladipat, Oleylerucat, Capryl/Caprinsäureester von gesättigten $C_{12}$ - $C_{18}$ Fettalkoholen, Myristylmyristat, Isoadipat, Isopropylpalmitat, Isopropylmyristat, Decyloleat, 2-Ethylhexyl-palmitat, Di-(2-ethylhexyl)-adipat, natürliche Öle (z.B. Erdnußöl, Jojobaöl usw.); als Antioxidantien: α-Tocopherol-acetat, Butylhydroxytoluol, Propylgallat; als Feuchthaltemittel/Penetrationsverstärker: mehrwertige Alkohole, wie z.B. Butandiol, Glycerol, Sorbitol, Pentaerythrol, Xylitol; als Konservierungsmittel: PHB-Ester, Benzylalkohol.

Die Herstellung der erfindungsgemäßen Zusammensetzungen kann auf eine für die Herstellung solcher Wasser-in-Öl-Emulsionen, z.B. in Form von Pasten, Salben, Emulsionen oder insbesondere in Form von W/O-Cremen, an sich übliche, allgemein bekannte Weise erfolgen. Dabei wird der Wirkstoff (Piroxicam) vorzugsweise vorab in einer Teilmenge der Ölkomponenten suspendiert. In einer zweckmäßigen Ausführungsform wird zuerst eine geeignete Fettphase hergestellt, in die dann der Wirkstoff (Piroxicam) unter Bildung der Ölphase eingearbeitet wird, was vorzugsweise unter Erwärmen erfolgt. Dann wird unter Rühren die für die gewünschte Darreichungsform geeignete Menge Wasser (Wasserphase), vorzugsweise in Gegenwart eines Emulgators zur Ausbildung einer stabilen Emulsion, zugegeben.

Wenn neutrale Konservierungsmittel verwendet werden, ist die wässerige Phase mit geeigneten Säuren auf einen sauren pH-Wert (pH = 2 bis 5) einzustellen.

Weil bei derartigen Emulsionen die Möglichkeit des Wachstums von Mikroorganismen besteht, ist es zweckmäßig, die erfindungsgemäßen Zusammensetzungen steril herzustellen und/oder zu konservieren. Aufgrund des Herstellungsverfahrens ist es möglich, die Sterilität der Zusammensetzung z.B. auf einfache Weise durch Erhitzen der Fett/Ölphase zu erreichen, weil für die Zusammensetzung und Wirksamkeit des erfindungsgemäßen Mittels gegebenenfalls stattfindende Phasenübergänge des Wirkstoffs von einer Phase in die andere keine so große Rolle spielen.

Gegenstand der Erfindung ist deshalb auch ein Verfahren gemäß Anspruch 21 zur Herstellung einer erfindungsgemäßen Zusammensetzung, das dadurch gekennzeichnet ist, daß man den Wirkstoff (Piroxicam) in die Ölphase einarbeitet, und diese dann mit der Wasserphase homogenisiert.

Eine zweckmäßige Ausgestaltung dieses Verfahrens ist Gegenstand von Anspruch 22.

Vorzugsweise wird das Piroxicam in einem mittelkettigen C 8 bis C 10-Triglycerid suspendiert, und diese Suspension dann in die geschmolzene Fettphase zur Bildung der Ölphase eingerührt. Die Zusammensetzung der Fettphase (Ölphase) und der Wasserphase entspricht dabei vorzugsweise den vorstehend für die Fettphase und die Wasserphase angegebenen Zusammensetzungen.

Die nachfolgenden Beispiele beschreiben erfindungsgemäße Zusammensetzungen und das Verfahren zu ihrer Herstellung, ohne die Erfindung auf diese Ausführungsformen zu beschränken. Vorstehend und nachfolgend beziehen sich Prozent- und Mengenangaben, wenn nicht anders angegeben, auf das Gewicht.

**BEISPIELE**

Beispiel 1

Herstellung einer W/O-Creme mit 0.5 Gew. % Piroxicam
Ansatz: 100 g

| Bestandteil | Menge in g |
|---|---|
| Polyethylenwachs-Dispersion (Polycos® 20) | 20.0 |
| Glycerin-Sorbitan-Oleostearat (Arlacel® 481) | 11.0 |
| 2-Octyl-dodecanol (Eutanol® G) | 5.0 |
| Butylhydroxyanisol (Embanox® BHA) | 0.01 |
| Mittelkettige Triglyceride (Miglyol® 812) | 5.0 |
| Piroxicam | 0.5 |
| Propylenglycol | 10.0 |
| Sorbinsäure | 0.01 |
| Magnesiumsulfat.7$H_2$O | 0.07 |
| Parfümöl | 0.09 |
| Ger. Wasser | 46.6 |

Die Herstellung der Creme erfolgt mit den folgenden Verfahrensschritten:

1. Die vorstehend genannten Bestandteile Polycos, Arlacel, Eutanol, Miglyol (4.0 g) und Embanox werden in einem VA-Topf unter Rühren bei 80°C aufgeschmolzen, wobei insbesondere auf ein vollständiges Aufschmelzen von Arlacel zu achten ist;

2. Die Sorbinsäure wird in Propylenglycol bei ca. 55°C gelöst;

3. das Wasser wird in einem VA-Topf auf 65 bis 70°C erwärmt, dann wird das Magnesiumsulfat gelöst, danach die unter 2 hergestellte Sorbinsäure-Lösung eingerührt; die Temperatur der so hergestellten Wasserphase soll 65°C betragen;

4. das Piroxicam wird mit einer hochtourigen Rühreinrichtung in Miglyol (1.0 g) suspendiert, und diese Suspension dann zur geschmolzenen Fettphase gegeben und kurz eingerührt;

5. die unter 3 hergestellte Wasserphase wird zur Fettphase zugegeben, emulgiert und homogenisiert;

6. es wird unter Vakuum kaltgerührt, bei 40°C dann das Parfüm eingerührt, dann ohne Vakuum kaltgerührt, und das Gefäß bei 28 bis 30°C entleert.

Eine bisher über 21 Monate laufende Stabilitätsprüfung an 2 Chargen zeigt eine leichte, im Rahmen der Spezifikation liegende Gelbfärbung. Gelbe Punkte, wie sie bei O/W-Cremes durch Bildung des gelben Piroxicam-Monohydrates auftreten, sind nicht zu erkennen. Die laufende Stabilitätsprüfung weist die W/O-Creme als stabile Arzneiform für Piroxicam aus.

Beispiel 2

Nach dem im Beispiel 1 beschriebenen Verfahren wurde die nachfolgende Zusammensetzung hergestellt.

Ansatz: 100 g

| Bestandteil | Menge in g |
|---|---|
| Wollwachs | 5.0 |
| Polyethylenwachs-Dispersion | 10.0 |
| Isopropylmyristat | 15.0 |
| Sorbitansesquioleat | 6.5 |
| Weißes Vaselin | 16.0 |
| Piroxicam | 1.0 |
| Sorbitol-Lösung 70% | 6.5 |
| Sorbinsäure | 0.15 |
| Ger. Wasser | 39.85 |

Eine Stabilitätsprüfung dieser Zusammensetzung zeigte gleich gute Ergebnisse wie die Zusammensetzung gemäß Beispiel 1.

Beispiel 3

Nach dem im Beispiel 1 angegebenen Verfahren wurde die folgende Zusammensetzung hergestellt:

Ansatz: 100 g

| Bestandteil | Menge in g |
|---|---|
| Wollwachs | 10.0 |
| Dünnfl. Paraffin | 15.0 |
| Polyethylen | 4.0 |
| Isopropylmyristat | 10.0 |
| Sorbitantrioleat | 6.5 |
| Weißes Vaselin | 10.0 |
| Piroxicam | 1.0 |
| Propylenglycol | 3.0 |
| Sorbinsäure | 0.15 |
| Ger. Wasser | 40.35 |

Die Stabilität dieser Zusammensetzung ist mit der der Zusammensetzungen der Beispiele 1 und 2 vergleichbar.

Die im Beispiel 1 hergestellte "Piroxicam-Creme" (Wirkstoffgehalt 0.5 Gew.%) wurde auf die antiphlogistische Wirkung geprüft, und mit einem Vergleichspräparat (Gel aus Propylenglycol, Ethyl- und Benzylalkohol mit 0.5 Gew. % Piroxicam) verglichen.

Es wurde die antiphlogistische Wirkung am UV-induzierten Erythem des Meerschweinchens untersucht (jeweils 10 Tiere/Gruppe).

Zur Auslösung des Erythems wurde die enthaarte Rückenhaut der Meerschweinchen 1 Minute mit UV-B-Licht (280 bis 315 nm bestrahlt). Anschließend wurden jeweils 50 mg der entsprechenden Präparate auf die bestrahlte Stelle aufgetragen.

Die 0.5 %-ige "Piroxicam-Creme" (erfindungsgemäß) hemmte die Ausprägung der Erytheme zwischen 27.8 % und 60 %, wobei die maximale Hemmwirkung 2 Stunden p.a. auftrat. Das Vergleichspräparat (Gel) wies Hemmwirkungen zwischen 13.3 % und 66.7 % auf, wobei das Maximum nach 1 Stunde p.a. verzeichnet wurde, wo auch in der Kontrollgruppe das Erythem noch nicht voll ausgeprägt war. Zu späteren Untersuchungszeitpunkten war die Hemmwirkung des Vergleichspräparates schwächer ausgeprägt als die der erfindungsgemäßen "Piroxicam-Creme".

Mit einer erfindungsgemäßen "Piroxicam-Creme" , die 1 Gew. % Wirkstoff (Piroxicam) enthielt, wurden ähnliche Hemmwirkungen (zwischen 22.2 % und 60.0 %) festgestellt, wobei das Maximum ebenfalls 2 Stunden p.a. registriert wurde.

Signifikante Verminderungen der Häufigkeit von Erythembefunden $\geqq$ 2 wurden in der Gruppe, die mit 0.5 %-iger "Piroxicam-Creme" behandelt wurde, 4 Stunden p.a., in der Gruppe, die mit 1 %-iger "Piroxicam-Creme" behandelt wurde 3 Stunden nach dem Auftragen im Vergleich zur Kontrollgruppe (Placebo-Gruppe) verzeichnet.

Die in der vorstehenden Untersuchung erzielten Ergebnisse zeigen deutlich die ausgeprägte antiphlogistische Wirkung der erfindungsgemäßen W/O-Cremes, wobei die antiphlogistische Wirkung des Vergleichspräparates gemäß dem Stande der Technik (Gel) im Hinblick auf eine Langzeitwirkung noch übertroffen wird.

Gegenstand der Erfindung ist deshalb auch eine erfindungsgemäße pharmazeutische Zusammensetzung als antirheumatisch, antiphlogistisch und/oder antiinflammatorisch wirkendes Arzneimittel zur Behandlung entsprechender Erkrankungen.

Die Art, Dosis und Häufigkeit der topischen Verabreichung richtet sich insbesondere nach der Schwere der Erkrankung und dem Allgemeinzustand des Patienten, aber auch nach dem Zustand und der Empfindlichkeit der Haut. In der Regel entspricht die Verabreichung den für derartige Zusammensetzungen üblichen Bedingungen.

## Patentansprüche

1. Pharmazeutische Zusammensetzung zur topischen Anwendung auf der Grundlage einer W/O-Emulsion, dadurch gekennzeichnet, daß sie in der Ölphase als Wirksubstanz Piroxicam enthält, und gegebenenfalls weitere Mittel zur Stabilisierung, Konservierung und/oder übliche pharmazeutische Hilfs- und/oder Trägerstoffe.

2. Zusammensetzung nach Anspruch 1, dadurch gekennzeichnet, daß die Ölphase eine Polyethylenwachs-Dispersion enthält.

3. Zusammensetzung nach Anspruch 1 oder 2, dadurch gekennzeichnet, daß die Ölphase mittelkettige Triglyceride und/oder 2-Octyldodecanol enthält.

4. Zusammensetzung nach einem der Ansprüche 1 bis 3, dadurch gekennzeichnet, daß sie Emulgatoren, Stabilisatoren, Antioxidantien, Lösungsvermittler, Penetrationsförderer, Penetrationsbeschleuniger, Konservierungsmittel, Emulsionsstabilisatoren und/oder Parfüms enthält.

5. Zusammensetzung nach Anspruch 4, dadurch gekennzeichnet, daß sie Glycerin-Sorbitan-Oleostearat, Butylhydroxyanisol, 2-Octyldodecanol, Propylenglycol und/oder Magnesiumsulfat-Heptahydrat enthält.

6. Zusammensetzung nach einem der Ansprüche 1 bis 5, dadurch gekennzeichnet, daß sie als Konservierungsmittel Sorbinsäure oder ein Gemisch aus Sorbinsäure und Propylenglycol enthält.

7. Zusammensetzung nach einem der Ansprüche 1 bis 6, dadurch gekennzeichnet, daß sie das Piroxicam in einer Menge von 0.1 bis 10 Gew. % enthält.

8. Zusammensetzung nach Anspruch 7, dadurch gekennzeichnet, daß sie das Piroxicam in einer Menge von 0.2 bis 5 Gew. %, und insbesondere in einer Menge von 0.5 bis 1 Gew. % enthält.

9. Zusammensetzung nach einem der Ansprüche 1 bis 8, dadurch gekennzeichnet, daß sie in Form einer W/O-Creme vorliegt.

10. Zusammensetzung nach einem der Ansprüche 1 bis 9, dadurch gekennzeichnet, daß sie als Salbengrundlage Polyethylenwachs-Dispersion, Wollwachs, weißes Vaselin, und/oder Polyethylen mit Paraffinöl enthält.

11. Zusammensetzung nach einem der Ansprüche 1 bis 10, dadurch gekennzeichnet, daß sie als Ölkomponente mittelkettige Triglyceride, 2-Octyldodecanol und/oder Isopropylmyristat enthält.

12. Zusammensetzung nach einem der Ansprüche 1 bis 11, dadurch gekennzeichnet, daß sie als Emulgator einen oder mehrere Emulgatoren mit einem HLB-Wert von 3 bis 6 enthält.

13. Zusammensetzung nach Anspruch 12, dadurch gekennzeichnet, daß sie als Emulgator Sorbitanfettsäureester und/oder Mischungen von Sorbitanfettsäureestern mit Glycerinfettsäureestern, Glycerinmonodifettsäureestern und/oder Glycerindifettsäureestern enthält.

14. Zusammensetzung nach einem der Ansprüche 9 bis 13, dadurch gekennzeichnet, daß sie ein Gemisch aus Polyethylenwachs-Dispersion, Glycerin-Sorbitan-Oleostearat, 2-Octyldodecanol und/oder mittelkettigen Triglyceriden, und Butylhydroxyanisol umfaßt.

15. Zusammensetzung nach einem der Ansprüche 9 bis 14, dadurch gekennzeichnet, daß sie 5 bis 30 % Salbengrundlage, 5 bis 25 % Emulgator, jeweils 0.5 bis 15 % einer oder mehrerer Ölkomponenten und 0.005 bis 0.15 % Antioxidantien, bezogen auf das Gewicht der Gesamtzusammensetzung, enthält.

16. Zusammensetzung nach einem der Ansprüche 9 bis 15, dadurch gekennzeichnet, daß das Gewichtsverhältnis von Ölphase zu Wasserphase ca. 3 zu 2 beträgt.

17. Zusammensetzung nach einem der Ansprüche 9 bis 16, dadurch gekennzeichnet, daß die Wasserphase Glycerol, Sorbitol, Pentaerythrol, Xylitol und/oder Propylenglycol enthält.

18. Zusammensetzung nach einem der Ansprüche 9 bis 17, dadurch gekennzeichnet, daß die Wasserphase Konservierungsmittel und/oder Stabilisatoren enthält.

19. Zusammensetzung nach Anspruch 18, dadurch gekennzeichnet, daß die Wasserphase Sorbinssäure und/oder Magnesiumsulfat-Heptahydrat enthält.

20. Zusammensetzung nach einem der Ansprüche 9 bis 19, dadurch gekennzeichnet, daß die Wasserphase 1 bis 20 % Feuchthaltemittel/Penetrationsverstärker, 0.01 bis 0.2 % Konservierungsmittel und/oder 0.25 bis 1.0 % Stabilisatoren, bezogen auf das Gewicht der Gesamtzusammensetzung, enthält.

21. Verfahren zur Herstellung einer Zusammensetzung nach einem der Ansprüche 1 bis 20, dadurch gekennzeichnet, daß man das Piroxicam in die Ölphase einarbeitet, und diese dann mit der Wasserphase homogenisiert.

22. Verfahren nach Anspruch 21 , dadurch gekennzeichnet, daß man das Piroxicam in der Ölkomponente suspendiert und diese Suspension in eine geschmolzene Fettphase zur Bildung der Ölphase einrührt.

23. Pharmazeutische Zusammensetzung nach einem der Ansprüche 1 bis 20 zur Verwendung als antirheumatisch, antiphlogistisch und/oder antiinflammatorisch wirkendes Arzneimittel.

**Claims**

1. Pharmaceutical composition for topical application on the basis of a W/O emulsion, characterised in that it contains piroxicam as active substance in the oil phase, and optionally further agents for stabilising, preserving and/or conventional pharmaceutical auxiliaries and/or excipients.

2. Composition according to claim 1, characterised in that the oil phase contains a polyethylene wax dispersion.

3. Composition according to claim 1 or 2, characterised in that the oil phase contains medium-chain triglycerides and/or 2-octyldodecanol.

4. Composition according to one of claims 1 to 3, characterised in that it contains emulsifiers, stabilisers, anti-oxidants, solubilisers, penetration promoters, penetration accelerators, preservatives, emulsion stabilisers and/or perfume.

5. Composition according to claim 4, characterised in that it contains glycerin-sorbitan-oleostearate, butylhydroxyanisole, 2-octyldodecanol, propylene glycol and/or magnesium sulphate heptahydrate.

6. Composition according to one of claims 1 to 5, characterised in that it contains sorbic acid or a mixture of sorbic acid and propylene glycol as preservative.

7. Composition according to one of claims 1 to 6, characterised in that it contains piroxicam in an amount from 0.1 to 10 wt.%.

8. Composition according to claim 7, characterised in that it contains piroxicam in an amount from 0.2 to 5 wt.%, and in particular in an amount from 0.5 to 1 wt.%.

9. Composition according to one of claims 1 to 8, characterised in that it exists in the form of an W/O cream.

10. Composition according to one of claims 1 to 9, characterised in that it contains polyethylene wax dispersion, wool wax, white Vaseline, and/or polyethylene with paraffin oil as ointment base.

11. Composition according to one of claims 1 to 10, characterised in that it contains medium-chain triglycerides, 2-octyldodecanol and/or isopropyl myristate as oil component.

12. Composition according to one of claims 1 to 11, characterised in that it contains one or more emulsifiers having an HLB value from 3 to 6 as emulsifier.

13. Composition according to claim 12, characterised in that it contains sorbitan fatty acid esters and/or mixtures of sorbitan fatty acid esters with glycerin fatty acid esters, glycerin monodifatty acid esters and/or glycerin difatty acid esters as emulsifier.

14. Composition according to one of claims 9 to 13, characterised in that it includes a mixture of polyethylene wax dispersion, glycerin-sorbitan-oleostearate, 2-octyldodecanol and/or medium-chain triglycerides, and butylhydroxyanisole.

15. Composition according to one of claims 9 to 14, characterised in that it contains 5 to 30 % of ointment base, 5 to 25 % of emulsifier, in each case 0.5 to 15 % of one or more oil components and 0.005 to 0.15 % of anti-oxidants, based on the weight of the total composition.

16. Composition according to one of claims 9 to 15, characterised in that the weight ratio of oil phase to water phase is about 3 to 2.

17. Composition according to one of claims 9 to 16, characterised in that the water phase contains glycerol, sorbitol, pentaerythrol, xylitol and/or propylene glycol.

18. Composition according to one of claims 9 to 17, characterised in that the water phase contains preservatives and/or stabilisers.

19. Composition according to claim 18, characterised in that the water phase contains sorbic acid and/or magnesium sulphate heptahydrate.

20. Composition according to one of claims 9 to 19, characterised in that the water phase contains 1 to 20 % of humectant/penetration enhancer, 0.01 to 0.2 % of preservative and/or 0.25 to 1.0 % of stabilisers, based on the weight of the total composition.

21. Process for producing a composition according to one of claims 1 to 20, characterised in that piroxicam is incorporated into the oil phase, and this is then homogenised with the water phase.

22. Process according to claim 21, characterised in that piroxicam is suspended in the oil component and this suspension is stirred into a molten fatty phase to form the oil phase.

23. Pharmaceutical composition according to one of claims 1 to 20 for use as an anti-rheumatic, anti-phlogistic and/or anti-inflammatory medicament.

## Revendications

1. Composition pharmaceutique pour application topique, à base d'une émulsion eau-dans-huile, caractérisée en ce qu'elle contient dans la phase huileuse, comme principe actif, du piroxicam, et, le cas échéant, des agents supplémentaires pour la stabilisation, la conservation et/ou des substances auxiliaires et/ou des supports pharmaceutiques habituels.

2. Composition selon la revendication 1, caractérisée en ce que la phase huileuse contient une dispersion de cire de polyéthylène.

3. Composition selon les revendications 1 ou 2, caractérisée en ce que la phase huileuse contient des triglycérines à chaîne moyenne et/ou du 2- octyldodécanol.

4. Composition selon l'une quelconque des revendications 1 à 3, caractérisée en ce qu'elle contient des émulsionnants, des stabilisants, des anti-oxydants, des agents de solubilisation, des agents favorisant la pénétration, des accélérateurs de pénétration, des agents conservateurs, des stabilisants d'émulsion et/ou des parfums.

5. Composition selon la revendication 4, caractérisée en ce qu'elle contient de l'oléostéarate de glycérol-sorbitane, du butylhydroxyanisol, du 2- octyldodécanol, du propylèneglycol et/ou de l'heptahydrate de sulfate de magnésium.

6. Composition selon l'une des revendications 1 à 5, caractérisée en ce qu'elle contient, comme agent conservateur, de l'acide sorbique ou un mélange d'acide sorbique et de propylèneglycol.

7. Composition selon l'une des revendication 1 à 6, caractérisée en ce qu'elle contient le piroxicam en une quantité de 0,1 à 10 % en poids.

8. Composition selon la revendication 7, caractérisée en ce qu'elle contient le piroxicam en une quantité de 0,2 à 5 % en poids et en particulier en une quantité de 0,5 à 1 % en poids.

9. Composition selon l'une des revendications 1 à 8, caractérisée en ce qu'elle est sous la forme d'une crème eau-dans-huile.

10. Composition selon l'une des revendications 1 à 9, caractérisée en ce qu'elle contient comme base pour pommade, une dispersion de cire de polyéthylène, de la lanoline, de la vaseline blanche et/ou du polyéthylène avec de l'huile de paraffine.

11. Composition selon l'une des revendications 1 à 10, caractérisée en ce qu'elle contient, comme constituant huileux, des triglycérides à chaîne moyenne, du 2-octyldodécanol et/ou du myristate d'isopropyle.

12. Composition selon l'une des revendications 1 à 11, caractérisée en ce qu'elle contient, comme émulsionnant, un ou plusieurs émulsionnants ayant une valeur d'équilibre hydrophile-lipophile de 3 à 6.

13. Composition selon la revendication 12, caractérisée en ce qu'elle contient comme émulsionnant des esters d'acide gras du sorbitane et/ou des mélanges d'esters d'acide gras du sorbitane avec des esters d'acides gras du glycérol, des monoesters de diacides gras du glycérol et/ou des diesters d'acides gras du glycérol.

14. Composition selon l'une des revendications 9 à 13, caractérisée en ce qu'elle comprend un mélange de dispersion de cire de polyéthylène, d'oléostéarate de glycérol/sorbitane, de 2-octyldodécanol et/ou de triglycérides à chaîne moyenne et de butylhydroxyanisole.

15. Composition selon l'une des revendications 9 à 14, caractérisée en ce qu'elle contient 5 à 30 % de base pour pommade, 5 à 25 % d'émulsionnant, à chaque fois 0,5 à 15 % d'un ou de plusieurs constituants huileux et 0,005 à 0,15 % d'antioxydants, par rapport au poids total de la composition.

16. Composition selon l'une des revendications 9 à 15, caractérisée en ce que le rapport pondéral de la phase huileuse à la phase aqueuse est d'environ 3 à 2.

17. Composition selon l'une des revendications 9 à 16, caractérisée en ce que la phase aqueuse contient du glycérol, du sorbitol, du pentaérythritol, du xylitol et/ou du propylèneglycol.

18. Composition selon l'une des revendications 9 à 17, caractérisée en ce que la phase aqueuse contient des agents conservateurs et/ou des stabilisants.

19. Composition selon la revendication 18, caractérisée en ce que la phase aqueuse contient de l'acide sorbique et/ou de l'heptahydrate de sulfate de magnésium.

20. Composition selon l'une des revendications 9 à 19, caractérisée en ce que la phase aqueuse contient 1 à 20 % d'agent de conservation de l'humidité/renforçateur de pénétration, 0,01 à 0,2 % de conservateur et/ou 0,25 à 1,0 % de stabilisants par rapport au poids total de la composition.

21. Procédé de préparation d'une composition selon l'une des revendications 1 à 20, caractérisé en ce qu'on incorpore le piroxicam à la phase huileuse, et en ce qu'on homogénéise alors celle-ci avec la phase aqueuse.

22. Procédé selon la revendication 21, caractérisé en ce qu'on met en suspension le piroxicam dans le constituant huileux et en ce qu'on délaye cette suspension dans une phase graisseuse fondue pour former la phase huileuse.

23. Composition pharmaceutique selon l'une des revendications I à 20, pour l'utilisation comme médicament ayant une action anti-rhumatismale, antiphlogistique et/ou anti-inflammatoire.